# EUROPEAN PATENT SPECIFICATION

(11) **EP 2 150 621 B1**
(45) Date of publication and mention of the grant of the patent: **21.11.2012**
(21) Application number: 08736053.3
(22) Date of filing: 10.04.2008
(51) Int. Cl.: C12P 19/04, C08L 1/02, A61L 27/20, B29C 67/00

(54) **Process for the production of a structure comprising crystalline cellulose**
Verfahren zur Herstellung einer Struktur umfassend kristalline Cellulose
Procédé pour la production d'une structure comprenant de la cellulose cristalline

(30) Priority: 10.04.2007 DE 102007016852
(43) Date of publication of application: 10.02.2010
(73) Proprietor: Bioregeneration GmbH, 85748 Garching (DE)
(72) Inventor: BERTHOLDT, Günter, 73092 Heiningen (DE)
(74) Representative: Keilitz, Wolfgang
(86) International application number: PCT/EP2008/054332
(87) International publication number: WO 2008/122661

(56) References cited:
- WO-A-2007/093445
- US-A- 5 506 607

## Description

### Background of the invention

The invention relates to a process for the production of a structure comprising crystalline cellulose according to the preamble of claim 1, the process comprising the use of a 3D printer and cellulose-forming organisms.

### Prior art

Structures of crystalline cellulose can be useful in numerous applications, for example as a support structure for culture of living cells *in vitro* ("tissue engineering") from cells which have been removed from an organism, in order to implant the cultured cells into the same organism and to obtain or re-establish a tissue function in this way. The tissues are preferably soft tissue, e.g. skin, muscle or fatty tissue, in contrast to hard or bone tissue.

A living tissue as a rule comprises a large number of specialized cells which influence each other through the aid of signal molecules, and it is presumed that cells orientate themselves within concentration gradients of low molecular weight substances and can behave in a particular manner. This is also referred to as "positional information". Between the cells of a tissue there are support structures, called extracellular matrix, which are made of macro-molecules released from the cells and which are stabilizing the cells in their particular position. If the extracellular matrix is destroyed and the cells become mixed up, normal, differentiated body cells are no longer able to reorganize themselves and to build up the lost structures again. However, it has been found that these cells can resume their normal function if they are brought into their original spatial relationship with the corresponding neighbouring cells.

A known solution approach attempts to imitate the extracellular matrix by culturing cells "three-dimensionally" within specific support structures, also called scaffolds. Scaffolds are usually foam-like porous structures of large internal surface area. The international laid-open specification WO 2006/096791 discloses the use of numerous currently available absorbable and non-absorbable synthetic polymers for the generation of so-called nanofilaments from which a layered scaffold is to be built up. The dependent German Patent Application 10 2007 006 843 describes support structures of crystalline cellulose which comprise colonizable hollow cavities e.g. in the form of branching tube systems which are similar to blood vessels. For the production of these support structures, a process is described in which a hollow mould is colonized with cellulose-forming bacteria of the strain Acetobacter xylinum. The tube system is created here with the aid of spiral wax wires, for example of summer wax, as spacers arranged parallel one above the other and/or side by side. After the mould has been filled with cellulose, the wax wires are removed by melting. Structures of crystalline cellulose can also be suitable as implants for mammals. The international laid-open specification WO 2006/61026 A1, for example, discloses a process for the production of a hollow body from microcrystalline cellulose of bacterial origin, which is said to be implantable into the carotid artery of a rat without an adverse action, such as foreign body reactions or formation of thrombi. The dependent German Patent Application 10 2007 006 844 describes the imitation of veins as hollow bodies from microcrystalline cellulose. For the production, a hollow mould with a mould core of a combination of thin metal plates with cast wax bodies and manually introduced recesses, which together form a mould core, is proposed, the mould core being surrounded by a mould shell. The hollow mould is colonized with cellulose-forming bacteria of the strain Acetobacter xylinum. The wax is then removed by melting and the thin metal plate is detached mechanically.

The production processes described here for hollow moulds are distinguished to a high degree by manual interventions. Furthermore, adaptation for precisely one body part is extremely expensive, since the hollow moulds often have to be modelled first for this case. To the extent that the mould core of wax is dissolved by melting, each structure is a unique item and can be reproduced to only a limited degree. In particular, no internal build-up or an internal build-up of only limited complexity can be produced in a hollow structure by processes of the prior art.

The US laid-open specification US 5,506,607 moreover discloses a 3D printer which builds up three-dimensional models layer for layer from a curing modelling material. The layers are formed by ejecting small drops of the modelling material in a liquid or flowable consistency from one or more nozzles on to a substrate, the nozzles and the substrate being movable in relation to one another in the X, Y and Z direction. The substrate and the nozzles are controlled by a computer such that they produce the individual layers in the X-Y plane. The nozzles or the substrate are furthermore shifted in the Z direction, so that the nozzles can produce layers following one another in the Z direction. In addition to the modelling material, which forms the actual model, a removable support material can be ejected, which supports otherwise unsupported parts of the model, e.g. the cross-bar of an H-shaped model.

### The problem on which the invention is based

The invention is based on the object of providing an improved process for the production of a structure comprising crystalline cellulose and such a structure. The invention is furthermore based on the object of providing a novel use of a 3D printer for the production of a structure comprising crystalline cellulose. The invention is furthermore based on the object of providing an improved process for the production of a hollow mould, the hollow mould and the use of a 3D printer for the production of the hollow mould.

### Solution according to the invention

To achieve the object, the invention teaches a process for the production of a structure comprising crystalline cellulose according to the features of claim 1, processes for the production of a hollow mould having the features of claims 10 or 11.

The structure according to the invention can advantageously be employed e.g. as an implant and/or for culture of living cells, in particular cells from mammals or humans.

It is an aspect of the present invention that the advantageous properties of 3D printers are utilized for the production of support structures. An achievable advantage of the present invention is therefore that manual production or processing steps on the hollow moulds are replaced by mechanical steps or dispensed with completely

It is an achievable advantage of the present invention that by the use of a hollow mould, a structure adapted to a particular purpose is planned or natural models are imitated. It is a further achievable advantage of the present invention that structures and hollow moulds according to the invention can be produced reproducibly by the use of a 3D printer. It can be a further advantage that structures according to the invention can also be realized with complicated moulds by means of the 3D printer.

It is an achievable advantage of the process according to the invention for the production of a structure that it can use the principle of the "lost mould", i.e. a mould can be created or employed which at least partly loses its shape in the step of removal of the mould.

### Structure and development of the solution according to the invention

Advantageous embodiments and developments, which can be employed individually or in combination with one another, are the subject matter of the dependent claims.

It is possible to produce only individual parts of the hollow mould, preferably one or more mould cores, or all the parts of the hollow mould by means of the 3D printer. 3D printers which are included according to the invention are also known to the person skilled in the art as "rapid prototyping systems". A 3D printer which is conventionally used in dentistry, such as is disclosed, for example, in the US patent US 5,506,607, can be employed as the 3D printer after appropriate adaptation. This also applies in particular to the individual embodiments disclosed therein of the preferred embodiments mentioned below for the 3D printer and its operating method.

The layers are preferably formed by ejecting small drops of the modelling material in liquid or flowable consistency from one or more nozzles on to a substrate or a previous layer. The substrate can also be a part of the hollow mould. It is then preferably laid into the printer at the start of the printing operation. Preferably, such a part of the mould can be made of the modelling material or another material. The nozzles and the substrate can preferably be moved relative to one another in the X, Y and Z direction, particularly preferably controlled by means of a data processing unit. In this context, the substrate and the nozzles can preferably be controlled such that they produce the layers in the X-Y plane. The nozzles or the substrate can furthermore preferably be shifted in the Z direction, so that the nozzles can produce the following layer.

In a preferred embodiment of the invention, at least one support is furthermore built up in layers from a preferably removable support material, in order to support unsupported regions of the (part) mould, e.g. the cross-bar of an H-shaped mould. The support material is preferably removed after the (part) mould has been finished by the 3D printer, e.g. by dissolving in a solvent or by melting, so that the (part) mould which remains can be used for culture of the cellulose.

Preferably, in the process according to the invention, initially one or more outer contours and optionally one or more inner contours of the (part) mould are constructed in each layer by arranging drops of the modelling material ejected in succession in rows. A drop is preferably still at least partly molten when it meets an adjacent drop, so that the drops can merge into one another. An inner space enclosed by one or more contours can be filled with modelling material or another material. It is also conceivable that grid-like support structures are constructed in such an inner space. In a preferred process, an ejected drop at least partly overlaps with an already previously ejected adjacent drop in the same layer. It may also be advantageous to construct walls of a double layer of drops arranged in rows. These measures can contribute towards improving the surface quality of the (part) mould.

In a conceivable embodiment of the invention, at least partly molten drops of the modelling material are ejected into a powder layer, preferably likewise of modelling material, so that powder particles are joined to one another to form the (part) mould.

In a preferred embodiment of the present invention, the hollow mould comprises at least one mould core. This mould core can imitate, for example, the hollow cavity of a vessel system though which the blood flows in the body of an organism. On the other hand, the hollow mould can also comprise only a mould shell. Preferably, the hollow mould comprises a mould shell, which imitates, for example, the outer demarcation of a vein or artery, and a mould core. A vessel system can be imitated with the aid of the mould core and mould shell in this way.

In one embodiment of the present invention, parts of the hollow mould, in particular one or more of the mould cores, can be deformed irreversibly in order to remove the hollow mould after culturing of the cellulose-forming bacteria. Preferably, the step of removal of the mould comprises at least partial melting of the mould core. During removal of the hollow mould, the mould core is preferably substantially removed, particularly preferably quantitatively, i.e. without residue.

The melting point of the modelling material is preferably above 28 °C, particularly preferably at or above 30 °C, particularly preferably at or above 60 °C. It is an achievable advantage of this embodiment of the invention that the part of the mould produced with the 3D printer remains stable during culture of the cellulose. In a preferred embodiment of the invention, the melting point of the modelling material is between 95 and 110 °C. It is an achievable advantage of this embodiment of the invention that the cellulose support structure is not damaged during melting of the part of the mould produced with the 3D printer.

In a preferred embodiment, the modelling material is hydrophobic. It is an aspect of this embodiment of the invention, which is utilized, that a hydrophobic material is repelled by the hydrophilic surface of the cellulose body. It is an achievable advantage of this embodiment of the invention that the modelling material can be removed substantially quantitatively during removal of the mould.

Thermoplastic wax material or polymer material, in particular, can be employed as the modelling material. In a particular embodiment, a modelling material which can be employed by the 3D printer as a mould-forming material is employed. In particular, the materials which are mentioned in the US patent US 5,506,607 in column 9, line 65 to column 10, line 40 (tables) can be employed. Further materials, to which reference is made by the abovementioned specification, are included according to the invention as materials for the production of a hollow mould by means of a 3D printer. It is also conceivable to employ polyvinyl alcohol (PVA) or the so-called "summer wax" known from dental technology as the modelling material. A preferred modelling material is InduraCast, obtainable from Solidscape Inc., Marimack, New Hampshire (NH), USA.

If a support material is employed in addition to the modelling material in the production of the (part) mould, the modelling material is preferably one of the materials proposed as a "modelling compound (MC)" in the US patent US 5,506,607, and the support material is one of the "support materials (SM)" proposed in this patent. A preferred modelling material is InduraCast InduraFill, obtainable from Solidscape Inc., Marimack, New Hampshire (NH), USA. The melting point of the support material is preferably below 110 °C, particularly preferably below 100 °C, particularly preferably below 80 °C. In a preferred embodiment of the invention, the melting point of the modelling material is between 49 and 70 °C. It is an achievable advantage of this embodiment of the invention that the support material can be removed from the modelling material by melting after finishing of the (part) mould.

In one embodiment of the present invention, the hollow mould comprises various materials. Thus, material combinations of the abovementioned materials and/or metal and/or glass and/or Teflon and/or ceramics can be used according to the invention.

In a further preferred embodiment, a nutrient liquid for culture of biological organisms is introduced into the hollow mould by means of the 3D printer during the build-up of the hollow mould in layers. In particular, the nutrient medium which is employed for culture of the cellulose-forming organisms is introduced. This can be advantageous in particular if certain regions of the hollow mould are poorly accessible.

The preferred structure has at least one undercut such that it cannot be removed from the hollow mould without at least a part of the mould being deformed. Preferably, the structure has in its inside at least one hollow cavity which is constructed as a spacer by a mould core of the mould. The mould core is preferably produced by means of the 3D printer. The hollow cavity can be constructed such that it is accessible from the outside only by passage through a narrow point, the cross-section of which is smaller than the cross-section of the hollow cavity.

In a preferred embodiment, the inner space of the hollow mould substantially has the shape of an elongated hollow body, e.g. in order to imitate, as the structure according to the invention by means of the hollow mould, a vessel through the lumen of which a medium, preferably a liquid medium, particularly preferably blood or other body fluids, can be passed. The mould core, which serves as a spacer for the lumen, preferably has a substantially circular cross-section. Preferably, the mould core comprises notches in order to form indentations, particularly preferably similarly to natural vein valves, by means of the hollow mould on the elongated hollow body, so that the flow of a medium which is passed through the elongated hollow body is decelerated more in a shut-off direction than in a direction opposite to the shut-off direction.

Preferred structures produced with the mould according to the invention have hollow cavities which are suitable for colonization with living cells. These hollow cavities are preferably formed by mould cores which have been produced with the 3D printer. In a preferred production process, the mould core has at least one strand which branches at at least one point. Particularly preferably, at least some of the branches run together again at another point in order to create a system of tubes which branch and run together again, e.g. similarly to a blood vessel system. The hollow body formed with the hollow mould according to the invention preferably has at least two openings through which the deformed, preferably molten mould core or its residues can leave the inside of the hollow body.

In a preferred embodiment of the invention, imaging methods serve to plan the hollow mould, preferably the part which is produced by means of the 3D printer, preferably the mould core. Tomographic methods are particular preferred, in particular x-ray tomography and/or positron emission tomography and/or nuclear spin tomography. A hollow mould adapted to a patient, e.g. for a body part to be replaced, in particular a blood vessel system or a vein valve system, can be created by this means. In particular, it is conceivable to record organs or parts of organs of the patient, e.g. sections of blood vessels, with imaging methods and to imitate them with the process according to the invention. The data determined with the imaging methods are converted into data which can be read by the 3D printer, in particular CAD data, and optionally modified. These data which can be read by 3D printers then serve for the build-up in layers and the imitation of the patient data.

The support structure preferably substantially comprises water and crystalline cellulose, particularly preferably microcrystalline cellulose such as is formed by the bacterium Acetobacter xylinum. This material contains less than 10 per cent of crystalline cellulose and the water is bonded partly and to different degrees to the microcrystalline cellulose. Crystalline cellulose has proved to be particularly tissue-friendly in experiments. The cellulose-forming organisms are preferably bacteria, particularly preferably bacteria of the strain Acetobacter xylinum. It is conceivable that other cellulose-forming microorganisms are also employed, such as e.g. suitable strains of Agrobacterium, Rhizobium, Sarcina, Pseudomonas, Achromobacter, Aerobacter and Zooglea. Since the genes of the cellulose-synthesizing enzyme complexes of Acetobacter xylinum are known, these could also be introduced into other microorganisms, such as e.g. Escherichia coli, by using known methods of molecular biology, as a result of which these organisms could also synthesize cellulose.

Various nutrient media have been described for culturing Acetobacter xylinum. A suitable medium which is often used is the medium of Schramm and Hestrin described in Biochemical Journal 58 of 1954, p. 345-352. A disadvantage of this medium can be that it is not precisely defined, since it contains yeast extract and peptone.

For carrying out the present invention, a completely synthetic medium is preferred, as described e.g. by Forng et al. in Applied and Environmental Microbiology of 1989, vol. 55, no. 5, p. 1317-1319. A disadvantage of this medium can be the somewhat slower growth of the bacteria.

It is also conceivable to use the so-called Kombucha tea fungus for carrying out the invention. This culture comprises, in addition to Acetobacter xylinum, numerous other organisms living in symbiosis, such as yeasts and bacteria, and can be maintained by a medium comprising only black tea and sucrose (100 g/l).

### Brief description of the figures

The invention is explained more closely with further details in the following with the aid of figures in the form of diagrams and embodiment examples.

The figures show:
- Fig. 1:: A diagram of a 3D printer used according to the invention.
- Fig. 2:: A perspective diagram of a hollow mould with a mould shell and mould core for carrying out the production process according to the invention for the structure.
- Fig. 3:: A diagram in cross-section of the hollow mould from Fig. 2 with an elongated hollow body which has not yet completely grown and indication of the direction of growth.
- Fig. 4:: A perspective diagram of a mould core for an elongated cellulose hollow body according to the invention with indentations on its inner wall.
- Fig. 5:: A diagram of an embodiment of the structure according to the invention as a hollow body which imitates the function of a vein valve.
- Fig. 6:: A perspective diagram of an embodiment of a structure according to the invention as a support structure for culture of living cells.
- Fig. 7:: A diagram in cross-section of a build-up by carrying out the production process according to the invention.

### Description with the aid of embodiment examples

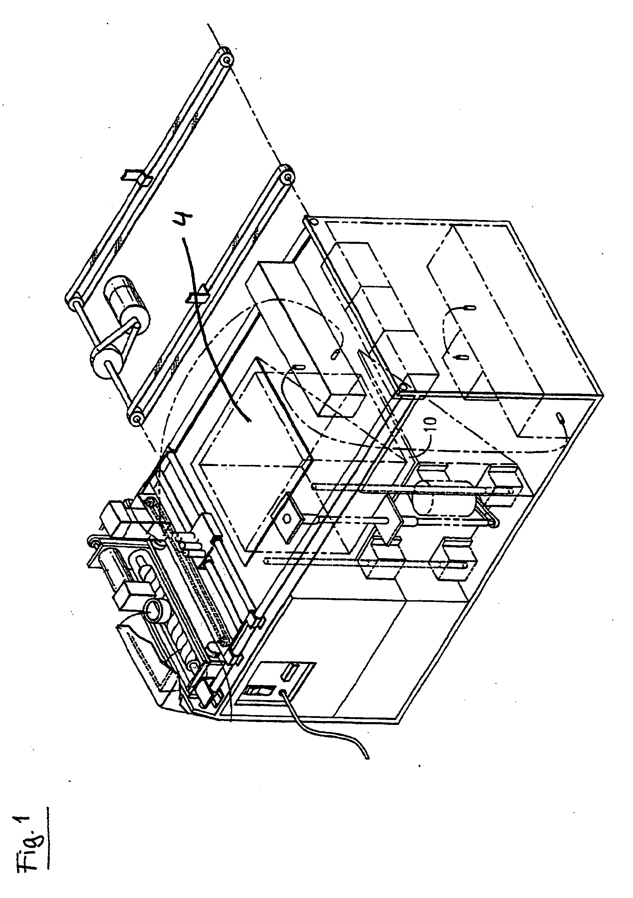

Figure 1 shows a 3D printer 1 from the prior art used in dentistry. The printer 1 substantially corresponds in construction and mode of functioning to the 3D printer disclosed in US 5,506,607. Parts 2a, 2b of the hollow mould are produced in layers with this by drops of a modelling material and a support material being arranged in rows on the substrate 4 or the previous layer. For application of the material, this is heated before the application and cures after the application. After all the material in one plane has been applied, material is again applied in the next higher plane. When this step is also concluded, the same procedure takes place with the plane lying above. A three-dimensional structure is formed in this way in time. InduraCast (Solidscape Inc., Marimack, NH, USA) was used as the modelling material and InduraFill (Solidscape Inc., Marimack, NH, USA) was used as the support material. After all the layers have been applied, the hollow mould is removed and the support material is removed by melting in accordance with the manufacturer's instructions, so that only the hollow mould of modelling material still remains.

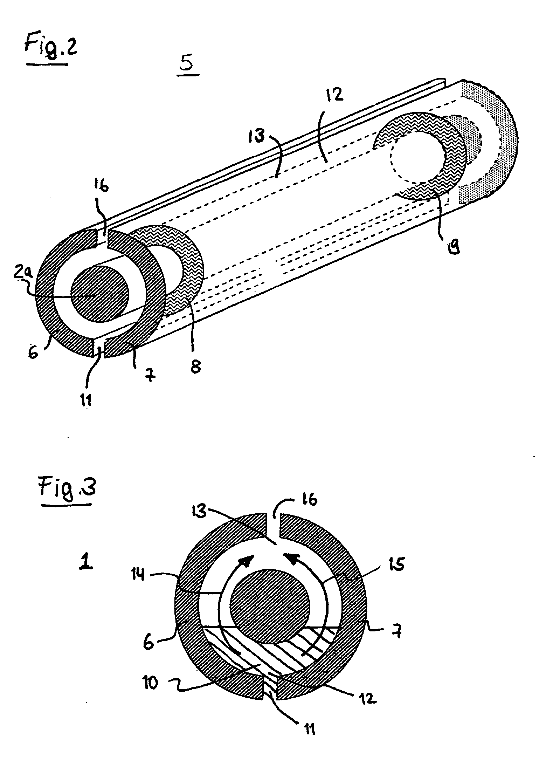

Figures 2 and 3 show a hollow mould 5 which has been formed by means of the process according to the invention. The half-shells 6 and 7, which form the mould shell, are made of glass, the spacers 8 and 9 are made of Teflon and the cylindrical mould core 2a has been produced by means of the 3D printer 1. Figure 3 show how the hollow mould 5 is filled with cellulose 10 by the cellulose-forming bacteria. Crystalline cellulose 10 grows through the lower opening 11 into the inner space perpendicularly to the longitudinal axis of the inner space from one longitudinal side 12 to the other longitudinal side 13 (shown by the arrows 14 and 15). At the same time, exchange of air with the environment of the hollow mould 5 can take place via a second opening 16, in particular in order to supply the bacteria with oxygen. Through the opening 11, the cellulose can transport from the outside the necessary nutrients from the growth medium to the organisms inside the hollow mould 5. When the cellulose 10 has arrived at the upper opening 16, the mould removal operation can be carried out. For this, the half-shells 6 and 7 are removed and the mould core 2a is melted. Thereafter, substantially the cellulose mould which has grown remains as a hollow body. It is to be noted at this point that in this case only the region within the two spacers 8 and 9 can be used, i.e. the entire hollow body must be supported. The hollow body formed can be used, for example, as a substitute for a blood vessel.

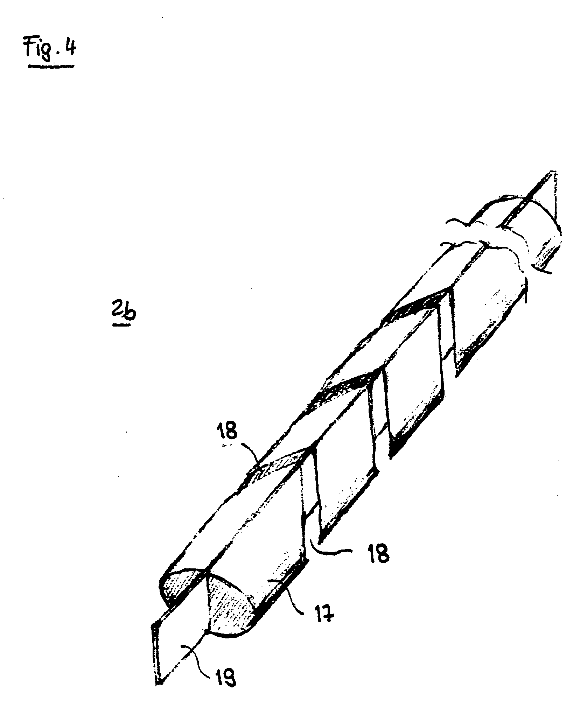

Figure 4 shows another mould core 2b which is likewise used with the mould shell shown in Figures 2 and 3. The mould core 2b substantially comprises a cylinder 17 which is produced with the 3D printer 1 and is provided with pairs of slits 18 which run together like an arrow in the middle of the mould core 2b. To stabilize the mould core 2b, a spring steel plate 19 is enclosed in the mould core 2b in the central plane. The slits 18, 19 extend over the entire width of the cylinder 17 up to the spring steel plate 19. During production of the mould core 2b, the cylinder half-shells are applied by the 3D printer to the steel plate 11 in layers.

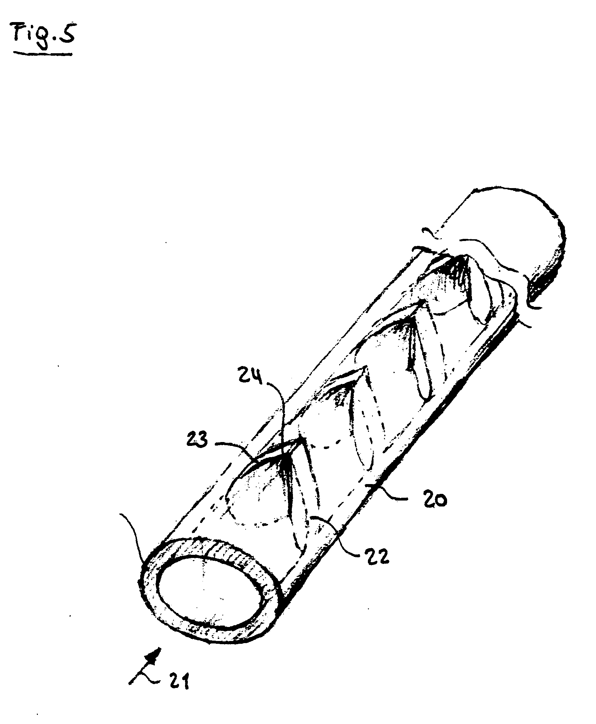

The mould core 2a with the mould shell is filled with cellulose 10 in the intermediate space between the mould shell and mould core 2a, as described above, and the mould is then removed. The hollow body 20 formed is shown in Figure 5. This hollow body 20 can imitate the function of vein valves. When a medium flows through the hollow body 20 against the direction of passage 21, the valve imitations 22, 23 which have been created by the slits 18, 19 open at the gap 24 which has been created by the plate 19 and allow the medium to flow. If the medium flows in the shut-off direction, the valve imitations 22, 23 are pressed against one another and the flow of the medium is substantially suppressed.

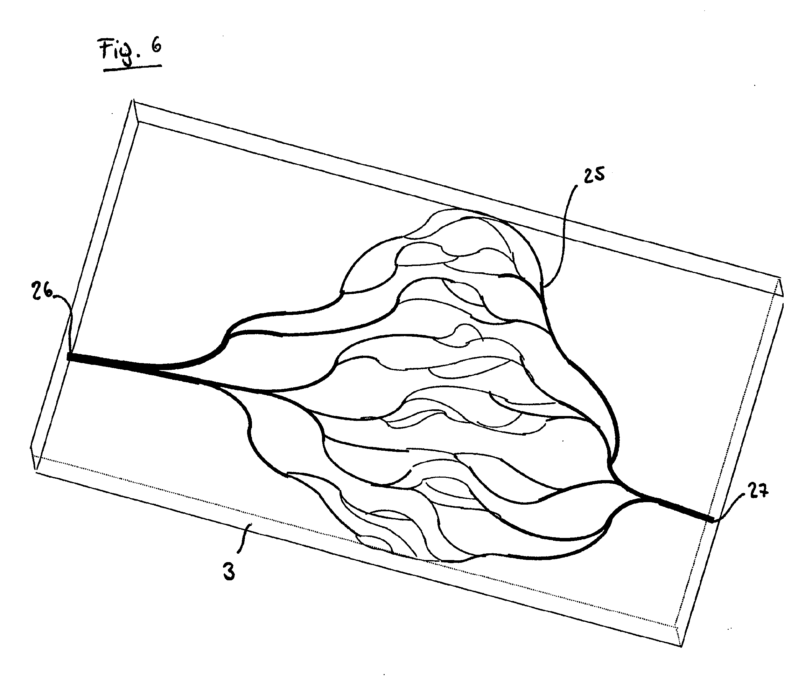

Fig. 6 shows an example of a support structure 3 which was created in the form of a network of strands which branch and run together again by means of a mould core produced with the 3D printer. After the cellulose has completely filled a hollow mould with the wax wire mesh as the mould core, the support structure 3 is removed and heated to 110 °C, in order to melt the mould core. The modelling material can substantially be removed completely from the support structure 3 in this way. A hollow cavity 25 of tubes which branch and run together again, similarly to a blood vessel system, remains. The hollow cavity 25 is connected to the outside of the support structure by an opening at two points. The points at which the hollow cavity 25 branches and the points at which the branches run together again are arranged between the two points 26, 27 at which the hollow cavity 25 is connected to the outside of the support structure 3 by openings.

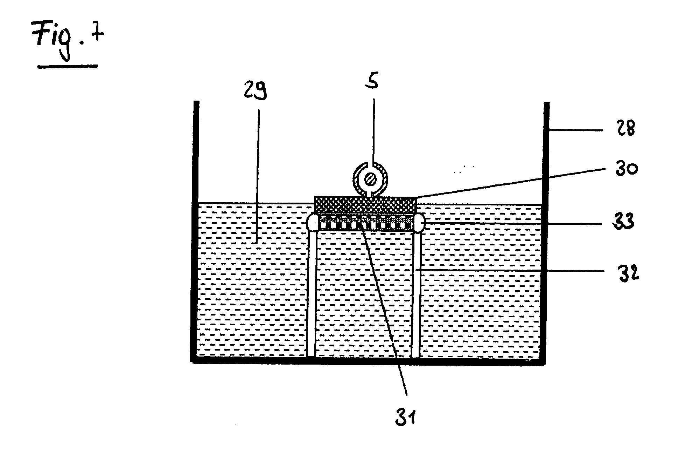

Fig. 7 shows a diagram of an embodiment example of an arrangement for carrying out the production process according to the invention for a support structure 2a, 2b. A sterile vessel 28 is filled with a sterile nutrient solution 29 comprising 20 g of glucose, 5 g of yeast extract, 5 g of bactopeptone, 2.7 g of sodium phosphate and 1.15 g of citric acid monohydrate, pH 6.0, and the nutrient solution is inoculated with a 3 day-old preculture from Acetobacter xylinum (e.g. Gluconacetobacter xylinum, DSM no. 2325, DSZM Braunschweig). When a layer 30 of cellulose about 3 mm thick has formed on the surface of the liquid after approx. 7 days, this is supported by a net 31 of Teflon (ePTFE expanded polytetrafluoroethylene, e.g. GLIDE dental floss, W. L. Gore and Associates Inc.) clamped in a glass frame 33 carried by supports 32 of glass. The hollow mould 5 is laid on the cellulose surface supported with the net 31 and culturing is carried out at 28 °C in an incubating cabinet.

Colonization of the hollow mould 5 by the bacteria and filling of this with cellulose as a rule takes 2 to 3 weeks. During this time it is to be ensured that medium 29 which has been used up or evaporated is replaced if appropriate. When the hollow mould 5 is completely filled with cellulose 10, the structure 2a, 2b, 3 is removed and heating to approx. 110 °C is then carried out, so that the mould core 2a, 2b melts and leaves behind hollow cavities 25 in the structure 2a, 2b. Heating at the same time serves to sterilize the structure.

## Claims

1. Process for the production of a structure comprising crystalline cellulose (10), the process comprising the steps of:
producing a hollow mould (5) by means of a 3D printer (1) the 3D printer building up at least a part (2a, 2b) of the hollow mould (5) in layers from a modelling material, and
culturing cellulose (10)-forming organisms at least partly in the hollow mould (5).

2. Process according to claim 1,
**characterized in that** the hollow mould (5) is at least partly irreversibly deformed.

3. Process according to one of the preceding claims,
**characterized in that** the hollow mould (5) comprises at least one mould core (2a, 2b).

4. Process according to claim 1 or 2,
**characterized in that** the hollow mould (5) comprises at least one mould shell.

5. Process according to one of the preceding claims,
**characterized in that** the hollow mould (5) comprises both at least one mould shell and a mould core (2a, 2b).

6. Process according to one of the preceding claims,
**characterized in that** the modelling material is at least partly hydrophobic.

7. Process according to one of the preceding claims,
**characterized in that** the modelling material comprises a thermoplastic wax or polymer.

8. Process according to one of the preceding claims
**characterized in that** during the build-up of the part (2a, 2b) of the hollow mould (5) in layers, the 3D printer (1) furthermore builds up at least one support from a support material in layers.

9. Process according to one of the preceding claims,
**characterized in that** a nutrient liquid is introduced into the hollow mould (5) by means of the 3D printer (1) for culture of the cellulose (10)-forming organisms.

10. Process for the production by means of a 3D printer (1) of a hollow mould (5) which is useable for culture of cellulose (10)-forming organisms,
**characterized in that** the hollow mould (5) comprises a mould core (2a, 2b) with strands which branch and run together again at another point, and the 3D printer (1) builds up at least the mould core (2a, 2b) of the hollow mould (5) in layers from a modelling material.

11. Process for the production of a hollow mould (5) which is useable for culture of cellulose (10)-forming organisms, the process comprising the steps of:
- Determining the hollow mould (5) by means of an imaging method and
- producing the hollow mould (5) by building up at least a part of the hollow mould (5) in layers from a modelling material by means of a 3D printer.

12. Process according to claim 11,
**characterized in that** tomographic methods serve as imaging methods.

## Patentansprüche

1. Verfahren zur Herstellung einer kristalline Cellulose (10) aufweisenden Struktur, umfassend die Schritte:
Herstellen einer Hohlform (5) mittels eines 3D-Druckers (1), wobei mit dem 3D-Drucker mindestens ein Teil (2a, 2b) der Hohlform (5) in Schichten aus einem Modelliermaterial aufgebaut wird, und
Kultivieren von Cellulose (10) bildenden Organismen, zumindest teilweise in der Hohlform (5).

2. Verfahren nach Anspruch 1,
**dadurch gekennzeichnet, dass** die Hohlform (5) wenigstens teilweise irreversibel verformt wird.

3. Verfahren nach einem der vorstehenden Ansprüche,
**dadurch gekennzeichnet, dass** die Hohlform (5) wenigstens einen Formkern (2a, 2b) aufweist.

4. Verfahren nach Anspruch 1 oder 2,
**dadurch gekennzeichnet, dass** die Hohlform (5) wenigstens eine Formhülle aufweist.

5. Verfahren nach Anspruch einem der vorstehenden Ansprüche,
**dadurch gekennzeichnet, dass** die Hohlform (5) sowohl wenigstens eine Formhülle und einen Formkern (2a, 2b) aufweist.

6. Verfahren nach einem der vorstehenden Ansprüche,
**dadurch gekennzeichnet, dass** das Modelliermaterial wenigstens teilweise hydrophob ist.

7. Verfahren nach einem der vorstehenden Ansprüche,
**dadurch gekennzeichnet, dass** das Modelliermaterial ein thermoplastisches Wachs oder Polymer aufweist.

8. Verfahren nach einem der vorstehenden Ansprüche,
**dadurch gekennzeichnet, dass** der 3D-Drucker (1) während des Aufbaus des Teils (2a, 2b) der Hohlform (5) in Schichten außerdem mindestens eine Stutze aus Stützmaterial in Schichten aufbaut.

9. Verfahren nach einem der vorstehenden Anspruche,
**dadurch gekennzeichnet, dass** mittels des 3D-Druckers (1) eine Nährflüssigkeit zum Kultivieren der Cellulose (10) bildenden Organismen in die Hohlform (5) eingebracht wird.

10. Verfahren zur Herstellung einer zum Kultivieren von Cellulose (10) bildenden Organismen geeigneten Hohlform (5) mittels eines 3D-Druckers (1),
**dadurch gekennzeichnet, dass** die Hohlform (5) einen Formkern (2a, 2b) mit sich verzweigenden und an anderer Stelle wieder zusammenlaufenden Strängen aufweist, und der 3D-Drucker (1) mindestens einen Teil des Formkerns (2a, 2b) der Hohlform (5) in Schichten aus einem Modelliermaterial aufbaut.

11. Verfahren zur Herstellung einer zum Kultivieren von Cellulose (10) bildenden Organismen geeigneten Hohlform (5), umfassend die folgenden Schritte:
- Bestimmen der Hohlform (5) mittels eines bildgebenden Verfahrens und
- Herstellen der Hohlform (5), indem mittels eines 3D-Druckers mindestens ein Teil der Hohlform (5) in Schichten aus einem Modelliermaterial aufgebaut wird.

12. Verfahren nach Anspruch 11,
**dadurch gekennzeichnet, dass** tomographische Verfahren als bildgebende Verfahren dienen.

## Revendications

1. Procédé de production d'une structure comprenant de la cellulose cristalline (10), le procédé comprenant les étapes de :
produire un moule creux (5) au moyen d'une imprimante 3D (1), l'imprimante 3D formant au moins une partie (2a, 2b) du moule creux (5) en couches à partir d'un matériau de modelage, et
cultiver des organismes formant de la cellulose (10) au moins partiellement dans le moule creux (5).

2. Procédé selon la revendication 1,
**caractérisé en ce que** le moule creux (5) est au moins partiellement déformé de manière irréversible.

3. Procédé selon la revendication 1 ou 2,
**caractérisé en ce que** le moule creux (5) comprend au moins un noyau de moule (2a, 2b).

4. Procédé selon la revendication 1 ou 2,
**caractérisé en ce que** le moule creux (5) comprend au moins une carcasse de moule.

5. Procédé selon l'une quelconque des revendications précédentes,
**caractérisé en ce que** le moule creux (5) comprend à la fois au moins une carcasse de moule et un noyau de moule (2a, 2b).

6. Procédé selon l'une quelconque des revendications précédentes,
**caractérisé en ce que** le matériau de modelage est au moins partiellement hydrophobe.

7. Procédé selon l'une quelconque des revendications précédentes,
**caractérisé en ce que** le matériau de modelage comprend une cire thermoplastique ou un polymère.

8. Procédé selon l'une quelconque des revendications précédentes,
**caractérisé en ce que** pendant la formation de la partie (2a, 2b) du moule creux (5) en couches, l'imprimante 3D (1) forme en outre au moins un support à partir d'un matériau de support en couches.

9. Procédé selon l'une quelconque des revendications précédentes,
**caractérisé en ce qu'**un liquide nutritif est introduit dans le moule creux (5) au moyen de l'imprimante 3D (1) pour la culture des organismes formant de la cellulose (10).

10. Procédé pour la production au moyen d'une imprimante 3D (1) d'un moule creux (5) qui peut être utilisé pour la culture d'organismes formant de la cellulose (10),
**caractérisé en ce que** le moule creux (5) comprend un noyau de moule (2a, 2b) avec des brins qui dérivent et repartent ensemble à un autre point, et l'imprimante 3D (1) forme au moins le noyau de moule (2a, 2b) du moule creux (5) en couches à partir d'un matériau de modelage.

11. Procédé pour la production d'un moule creux (5) qui peut être utilisé pour la culture d'organismes formant de la cellulose (10), le procédé comprenant les étapes de :
- déterminer le moule creux (5) au moyen d'un procédé d'imagerie et
- produire le moule creux (5) en formant au moins une partie du moule creux (5) en couches à partir d'un matériau de modelage au moyen d'une imprimante 3D.

12. Procédé selon la revendication 11,
**caractérisé en ce que** des procédés tomographiques servent de procédés d'imagerie.
